# EUROPEAN PATENT APPLICATION

(11) **EP 3 360 543 A1**
(43) Date of publication of application: **15.08.2018**
(21) Application number: 18156512.8
(22) Date of filing: 13.02.2018
(51) Int. Cl.: A61K 9/20, A61K 31/496

(54) **PHARMACEUTICAL COMPOSITIONS OF VILAZODONE HYDROCHLORIDE**

(30) Priority: 13.02.2017 TR 201702104
(71) Applicant: Sanovel Ilac Sanayi ve Ticaret A.S., 34460 Istanbul (TR)
(72) Inventor: TÜRKYILMAZ, Ali, 34460 Istanbul (TR); YILDIRIM, Ediz, 34460 Istanbul (TR); KÖKSAL UZUN, Selin, 34460 Istanbul (TR)
(74) Representative: Sevinç, Erkan

(57) **Abstract**

The present invention relates to a pharmaceutical compositions comprising vilazodone hydrochloride and at least one pharmaceutically acceptable excipient.

## Description

### Field of Invention

The present invention relates to a pharmaceutical compositions comprising vilazodone hydrochloride and at least one pharmaceutically acceptable excipient.

### Background of the Invention

Vilazodone is used to treat depression. It is an SSRI (selective serotonin reuptake inhibitor) and partial serotonin receptor agonist. It works by helping to restore the balance of certain natural substances in the brain (neurotransmitters such as serotonin). This medication may improve your mood, sleep, appetite, and energy level and may help restore your interest in daily living.

The chemical designation of vilazodone is I-[4-(5-cyanoindol-3-yl) butyl]-4-(2-carbamoyl-benzofuran-5-yl)-piperazine hydrochloride. Its chemical structure is shown in the Formula-I.

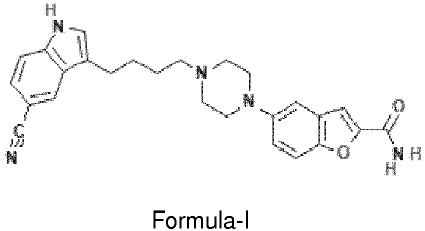

Vilazodone (commercially known as Viibryd®) is presently marketed as immediate-release tablets. Current guidelines for use of vilazodone in treatment of major depressive disorder recommends that vilazodone be administered at a starting dose of 10 mg once daily for seven days, followed by 20 mg once daily for additional seven days and then increased to 40 mg once daily.

Vilazodone hydrochloride is first disclosed in U.S. Patent No. 5,532,241.

Prior art shows that U.S. Patent No. 7,834,020 discloses fifteen crystalline modifications of vilazodone hydrochloride designated as Form I through Form XV. It also discloses conventional forms of administration such as tablets and capsules. PCT Publication No. WO 2012/131706 discloses the amorphous form of vilazodone hydrochloride. Further, PCT Publication No. WO 2013/078361 discloses several other polymorphic forms of vilazodone hydrochloride.

There still remains a need in the prior art to provide improved pharmaceutical compositions of viladozone hydrochloride, having a high solubility, dissolution rate, and therefore a high bioavailability and a long-term stability.

The patent application WO2015019237 A1 discloses a formulation, comprising micronized particles of vilazodone and processes for their preparation. It further relates to a method of treating and/or preventing central nervous system disorders by administering said pharmaceutical compositions. At this application, vilazodone hydrochloride has a d (0.5) particle size smaller than 20 µm and this cause the problem of solubility of vilazode hydrochloride, its flowability and its compressibility.

In comparison to the prior art, at present invention, vilazodone hydrochloride has a d (0.5) particle size not less than 33 µm in order to overcomes the described problems in prior art. This invention provide surprisingly better solubility and compressibility.

In comparison to the prior art, according to present invention crospovidone is used as disintegrant. It presents in amounts ranging from about 2.0% w/w to about 10.5% w/w. This certain ratio helps to achieve the desired release profile of the drug. Also, it stabilizes the active pharmaceutical ingredient which does not degrade to unwanted impurities.

### Detailed description of the Invention

The main object of the present invention is to provide a formulation with high stability, having desired level of dissolution rate and compressibility which overcomes the above described problems in the prior art and have additive advantages over them.

A further object of the present invention is to stabilize the active pharmaceutical ingredient which does not degrade to unwanted impurities.

Another object of the present invention is to obtain a good selection of excipients in a certain ratio which provides high bioavailability and dissolution, thus very important.

Another object of the present invention is to provide a vilazodone hydrochloride tablet comprising at least one film coating to protect the pharmaceutical composition against the moisture to maintain the stability.

As used here in, 'particle size' means the cumulative volume size distrubition as tested by any conventionally accepted method such as the laser diffraction method (i.e. malvern analysis). The term d (0.1) means, the size at which 10% by volume of the particles are finer and d (0.5) means the size at which 50% by volume of the particles are finer and d (0.9) means the size at which %90 by volume of the particles are finer.

According to this embodiment, the solid oral pharmaceutical composition comprises vilazodone hydrochloride which has a d (0.5) particle size not less than 33 µm, preferably d (0.5) particle size between 33 µm and 45 µm, between 45 µm and 75 µm to provide surprisingly better solubility and compressibility.

According to this embodiment, the solid oral pharmaceutical composition comprises vilazodone hydrochloride which has a d (0.5) particle size between 80 µm and 100 µm, between 100 µm and 120 µm, between 120 µm and 140 µm, between 140 µm and 160 µm, between 160 µm and 180 µm, between 180 µm and 200 µm, between 200 µm and 220 µm, between 220 µm and 240 µm, between 240 µm and 260 µm, between 260 µm and 280 µm, between 280 µm and 300 µm.

At present invention, vilazodone hydrochloride has a d (0.1) particle size not less than 9 µm, preferably d (0.1) particle size between 11 µm and 15 µm.

At present invention, vilazodone hydrochloride has a d (0.1) particle size between 19 µm and 30 µm, between 30 µm and 50 µm, between 50 µm and 70 µm, between 70 µm and 90 µm, between 90 µm and 110 µm, between 110 µm and 140 µm, between 140 µm and 160 µm, between 160 µm and 180 µm, between 180 µm and 200 µm, between 200 µm and 220 µm, between 220 µm and 240 µm, between 240 µm and 260 µm, between 260 µm and 280 µm, between 280 µm and 300 µm.

At present invention, vilazodone hydrochloride has a d (0.9) particle size not less than 65 µm, preferably it has a d (0.9) particle size between 65 µm and 90 µm, between 90 µm and 110 µm, between 110 µm and 140 µm.

At present invention, vilazodone hydrochloride has a d (0.9) particle size not less than 65 µm, preferably it has a d (0.9) particle size between 210 µm and 240 µm, between 240 µm and 260 µm, between 260 µm and 280 µm, between 280 µm and 300 µm, between 300 µm and 320 µm, between 320 µm and 340 µm, between 340 µm and 360 µm, between 360 µm and 380 µm, between 380 µm and 400 µm.

According to this embodiment, the total amount of vilazodone hydrochloride in the composition is 2.0% to 45.0% by weight further comprising crospovidone as disintegrant.

According to this embodiment, the total amount of vilazodone hydrochloride in the composition is 6.0% to 18.0% by weight further comprising crospovidone as disintegrant.

Suitable disintegrants are selected from group comprising crospovidone, ion exchange resin, hydroxypropylcellulose, croscarmellose sodium, starches, pectins, alginates, magnesium silicate, aluminum silicate, microcrystalline cellulose, sodium starch glycolate or mixtures thereof, preferably the disintegrant is crospovidone. It is present in amounts ranging from 2.0% w/w to 10.5% w/w in the composition.

The ratio of crospovidone to the other excipients are very important for this invention to improve the quality of the film-coated tablet, since it is known that disintegrants have tablet quality deteriorating properties, such as causing tablet hardness reductions and tablet surface roughness as a result of moisture absorption, and worsening the mouth touch due to a feeling of dryness as a result of saliva absorption. Regarding this knowledge, in this invention, crospovidone is used in minimal ratios as given above as the single disintegrant.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation is developed. The formulations should have no physicochemical incompatibility between the drug and the excipients.

According to one embodiment of the present invention, the composition further comprises at least one pharmaceutically acceptable excipient selected from fillers, binders, lubricants, glidants or mixtures thereof.

Suitable fillers or binders are selected from the group comprising lactose monohydrate, microcrystalline cellulose, lactose anhydrous, pregelatinized starch, calcium carbonate, calcium phosphate dibasic, calcium phosphate tribasic, calcium sulphate, kaolin, starch or mixtures thereof, preferably fillers or binders are lactose monohydrate and microcrystalline cellulose. It is present in amounts ranging from 10% w/w to 90% w/w in the composition.

According to this embodiment of the present invention, microcrystalline cellulose is present in amounts ranging from 5.0% to 40% in the composition.

According to this embodiment of the present invention, crospovidone is very effective excipient in the preparation of the tablet. It has been surprisingly found that the object of the present invention is achieved when crospovidone and microcrystalline cellulose are used in a certain ratio. Consequently, the ratio of microcrystalline cellulose to crospovidone may be in the range of 0.01-10.0 by weight, preferably the ratio is 1.0-6.0. Said ratio provides adequate disintegrating properties for the tablets of the present invention but also stabilizes the active pharmaceutical ingredient which does not degrade to unwanted impurities.

Suitable lubricants are selected from group comprising magnesium stearate, stearic acid, calcium stearate, zinc stearate, sodium stearyl fumarate, hydrogenated castor oil, hydrogenated vegetable oil, light mineral oil, mineral oil, polyethylene glycol, sodium benzoate or mixtures thereof, preferably lubricant is magnesium stearate. It is present in amounts ranging from 0.01% w/w to 10% w/w in the composition.

Suitable glidants are selected from group comprising colloidal silicon dioxide, water soluble excipient, hydrophilic polymers, silicon dioxide or mixtures thereof, preferably glidant is colloidal silicon dioxide. It is present in amounts ranging from 0.01% w/w to 10% w/w in the composition.

In one embodiment of the present invention, the solid oral pharmaceutical composition is in the form of film-coated tablet, coated tablet, trilayer tablet, bilayer tablet, multilayer tablet, orally disintegrating tablet, mini tablet, pellet, buccal tablet, sublingual tablet, effervescent tablet, immediate release tablet, modified release tablet, pill, capsule, oral granule or powder. Preferably, the pharmaceutical composition is in the form of film-coated tablet.

According to this embodiment, the solid oral pharmaceutical composition is in the form of a film-coated tablet which comprises polyvinyl alcohol, titanium dioxide, polyethylene glycol, talc, coloring agent or mixtures thereof.

Suitable coloring agents are selected from group comprising FD&C Blue, FD&C Yellow, FD&C Red, Quinoline yellow aluminium lake or mixtures thereof.

According to this embodiment, the solid oral pharmaceutical composition comprising 40 mg vilazodone hydrochloride has a hardness between 140 N and 180 N, between 180 N and 250 N, 20 mg vilazodone hydrochloride which has a hardness between 120 N and 280 N, 10 mg vilazodone hydrochloride which has a hardness between 90 N and 250 N because it is not brittle easily. Thus, provides high chemical and mechanical stability.

In one embodiment of the present invention, the solid oral pharmaceutical composition comprises 1.0-70.0% by weight of vilazodone hydrochloride, 25-80.0% by weight of lactose monohydrate, 5.0-40.0% by weight of microcrystalline cellulose, 2.0-10.5% by weight of crospovidone, 0.01-10.0% by weight of magnesium stearate, 0.01-10.0% by weight of colloidal silicon dioxide, 1.0 - 5.0% by weight of coating of the composition.

In one embodiment of the present invention, the solid oral pharmaceutical composition comprising vilazodone hydrochloride is prepared by direct compression method.

According to this embodiment, a process for preparing the solid oral pharmaceutical composition comprises the following steps:
a) sieving vilazodone hydrochloride, lactose monohydrate, microcrystalline cellulose, crospovidone and colloidal silicon dioxide and mixing them for 15 minutes
b) adding magnesium stearate and mixing the total mixture for 5 minutes
c) compressing the total mixture into tablets
d) coating with film coating the tablet, which comprising polyvinyl alcohol, titanium dioxide, polyethylene glycol, talc, coloring agent.

### Example

This example describes the solid oral pharmaceutical composition comprising vilazodone hydrochloride. The active ingredient and excipients of the composition are given below:

### Example 1: The solid oral pharmaceutical composition of vilazodone hydrochloride

| **Content** | Amount (% by weight of the total) |
|---|---|
| vilazodone hydrochloride | 6.0 - 18.0 |
| lactose monohydrate | 40.0 - 70.0 |
| microcrystalline cellulose | 10.0 - 30.0 |
| crospovidone | 3.0 - 10.0 |
| magnesium stearate | 0.1 - 8.0 |
| colloidal silicon dioxide | 0.05 - 8.0 |
| coating | 1.0 - 5.0 |

| **Coating Material Ingredients** | **Amount (% by weight of the coating)** |
|---|---|
| polyvinyl alcohol | 30.0 - 50.0 |
| titanium dioxide | 17.0 - 35.0 |
| talc | 8.0 - 25.0 |
| polyethylene glycol | 12.0 - 32.0 |
| coloring agent | 0.01- 5.0 |

### Process for example

A process for preparing the solid oral pharmaceutical composition in example 1 comprises the following steps:
a) sieving vilazodone hydrochloride, lactose monohydrate (spray-dried 11SD), microcrystalline cellulose Ph 102, crospovidone CL and colloidal silicon dioxide and mixing them for 15 minutes
b) adding magnesium stearate and mixing the total mixture for 5 minutes
c) compressing the total mixture into tablets
d) coating with film coating the tablet, which comprising polyvinyl alcohol, titanium dioxide, polyethylene glycol, talc, coloring agent.

## Claims

1. The solid oral pharmaceutical composition comprising vilazodone hydrochloride having a d (0.5) particle size not less than 33 µm.

2. The solid oral pharmaceutical composition according to claim 1, wherein the d (0.5) particle size of vilazodone hydrochloride is between 33 µm and 75 µm.

3. The solid oral pharmaceutical composition according to claim 2, wherein the d (0.1) particle size of vilazodone hydrochloride is not less than 9 µm.

4. The solid oral pharmaceutical composition according to claim 3, wherein the d (0.1) particle size of vilazodone hydrochloride is between 11 µm and 15 µm.

5. The solid oral pharmaceutical composition according to claim 4, wherein the d (0.9) particle size of vilazodone hydrochloride is not less than 65 µm.

6. The solid oral pharmaceutical composition according to claim 5, wherein the d (0.9) particle size of vilazodone hydrochloride is between 65 µm and 140 µm.

7. The solid oral pharmaceutical composition comprising vilazodone hydrochloride whose total amount in the composition is 2.0% to 45.0% by weight further comprising crospovidone as disintegrant.

8. The solid oral pharmaceutical composition according to claim 7, wherein the total amount of the vilazodone hydrochloride in the composition is 6.0% to 18.0% by weight.

9. The solid oral pharmaceutical composition according to the claim 8, wherein crospovidone is present in amounts ranging from 2.0% w/w to 10.5% w/w in the composition.

10. The solid oral pharmaceutical composition according to any preceding claim, further comprising fillers or binders which are selected from the group comprising lactose monohydrate, microcrystalline cellulose, lactose anhydrous, pregelatinized starch, calcium carbonate, calcium phosphate dibasic, calcium phosphate tribasic, calcium sulphate, kaolin, starch or mixtures thereof, preferably fillers or binders are lactose monohydrate and microcrystalline cellulose.

11. The solid oral pharmaceutical composition according to the claim 10, wherein microcrystalline cellulose is present in amounts ranging from 5.0% to 40% w/w in the composition.

12. The solid oral pharmaceutical composition according to any preceding claims, wherein the weight ratio of microcrystalline cellulose to crospovidone is 0.01-10.0, preferably the ratio is 1.0-6.0.

13. The solid oral pharmaceutical composition according to any preceding claims, wherein the composition is in the form of film-coated tablet, coated tablet, trilayer tablet, bilayer tablet, multilayer tablet, orally disintegrating tablet, mini tablet, pellet, buccal tablet, sublingual tablet, effervescent tablet, immediate release tablet, modified release tablet, pill, capsule, oral granule or powder, preferably in the form of a film-coated tablet.

14. The solid oral pharmaceutical composition according to any preceding claim, wherein pharmaceutical composition comprises 40 mg vilazodone hydrochloride which has a hardness between 140 N to 250 N, 20 mg vilazodone hydrochloride which has a hardness between 120 N to 280 N, 10 mg vilazodone hydrochloride which has a hardness between 90 N to 250 N.

15. The solid oral pharmaceutical composition according to any preceding claims, wherein the composition comprises;
1.0-70.0% by weight of vilazodone hydrochloride,
25-80.0% by weight of lactose monohydrate,
5.0-40.0% by weight of microcrystalline cellulose,
2.0-17.0% by weight of crospovidone,
0.01-10.0% by weight of magnesium stearate,
0.01-10.0% by weight of colloidal silicon dioxide
1.0 - 5.0% by weight of coating in the composition.

16. A process for preparing the solid oral pharmaceutical composition according to claim 15, the process comprises the following steps:
a) sieving vilazodone hydrochloride, lactose monohydrate, microcrystalline cellulose, crospovidone and colloidal silicon dioxide and mixing them for 15 minutes
b) adding magnesium stearate and mixing the total mixture for 5 minutes
c) compressing the total mixture into tablets
d) coating with film coating the tablet, which comprising polyvinyl alcohol, titanium dioxide, polyethylene glycol, talc, coloring agent.
